# EUROPÄISCHE PATENTSCHRIFT

(11) **EP 1 242 825 B1**
(45) Veröffentlichungstag und Bekanntmachung des Hinweises auf die Patenterteilung: **09.03.2005**
(21) Anmeldenummer: 00992093.5
(22) Anmeldetag: 27.12.2000
(51) Int. Cl.: G01N 33/92, C07K 14/775, A61K 35/14, C07K 1/36, G01N 33/50, G01N 33/68

(54) **GEWINNUNG VON LIPOPROTEINEN AUS KÖRPERFLÜSSIGKEITEN**
EXTRACTION OF LIPOPROTEINS FROM BODY FLUIDS
EXTRACTION DE LIPOPROTEINES CONTENUES DANS DES LIQUIDES ORGANIQUES

(30) Priorität: 28.12.1999 DE 19963420
(43) Veröffentlichungstag der Anmeldung: 25.09.2002
(73) Patentinhaber: SeBo GmbH, 64711 Erbach (DE)
(72) Erfinder: BOOS, Karl-Siegfried, 82131 Gauting (DE); SEIDEL, Dietrich, 82340 Feldafing (DE)
(74) Vertreter: Weiss, Wolfgang, Dipl.-Chem. Dr.
(86) Internationale Anmeldenummer: PCT/EP2000/013294
(87) Internationale Veröffentlichungsnummer: WO 2001/048476

(56) Entgegenhaltungen:
- WO-A-96/41198
- WO-A-98/07751
- US-A- 5 089 602
- DATABASE HTTP://BIOCHEM.ROCHE.COM [Online] Roche Molecular Biochemicals; 22. Dezember 1999 (1999-12-22) "h-Lipoprotein (a) ELISA" XP002175868
- ALBERTS B ET AL.: "Molekularbiologie der Zelle" 1995 , VCH , WEINHEIM, DE XP002175867 Seite 185; Tabelle 4.3
- GROSS E. ET AL: 'Isolation of Lipoprotein(a) Using the Regenerate of a Dextran Sulfate Cellulose LDL Apheresis System' PROTEIN EXPRESSION AND PURIFICATION Bd. 5, 1994, Seiten 112 - 117

## Beschreibung

Die Erfindung betrifft ein Verfahren zur Gewinnung von Lipoproteinen aus Körperflüssigkeiten, insbesondere aus Blut, Blutplasma oder Serum. Die Lipoproteine werden in nativer, bzw. biologisch weitestgehend aktiver Form gewonnen und können beispielsweise zur Herstellung von Kontrollproben oder Standards für diagnostische Tests oder zur Herstellung von hochkonzentrierten Infusions-/Nähr- oder/und Inkubationslösungen für therapeutische oder/und biologische Zwecke eingesetzt werden.

Die Qualitätssicherung im medizinischen Laboratorium soll in erster Linie dem Wohle der Patienten dienen. Ihnen sollte der höchst mögliche Qualitätsstandard bei Laboruntersuchungen gewährleistet werden, um Fehlzuordnungen und daraus resultierende Diagnose- und Therapiefehler zu vermeiden. Darüber hinaus dient die Qualitätssicherung der Laborergebnisse dem Schutz des Laborpersonals und des verantwortlichen Arztes bei etwa strittigen Fragen aufgrund falscher Analysenergebnisse oder Zuordnungen. Die Qualitätssicherung für medizinische Laboratorien ist durch die Richtlinien der Bundesärztekammer für die Bundesrepublik Deutschland (RILI-BÄK) sowie vergleichbar durch entsprechende Verordnungen in den meisten anderen Ländern festgelegt. Entsprechend ist die RILI-BÄK ein Bestandteil der Eichordnung und hat dadurch gesetzlichen Charakter.

Die Qualitätskontrolle im medizinischen Laboratorium soll folgenden Aufgaben gerecht werden:
1. Der Überwachung zufälliger Meßabweichungen (Präzisionskontrolle)
2. Der Überwachung systematischer Meßabweichungen (Richtigkeitskontrolle)
3. Der Kontrolle von Matrix-Einflüssen auf Richtigkeit, Präzision und Spezifität der Messung.
4. Der Erkennung von Trends (z.B. Stabilität der Meßsysteme).

Die Qualitätssicherung nach diesen Richtlinien umfaßt die laborinterne Qualitätskontrolle (Präzisions- und Richtigkeitskontrolle) sowie die externe Richtigkeitskontrolle in Form von Ringversuchen. Sie muß- soweit technisch möglich - für alle untersuchten klinisch-chemischen Parameter erfolgen.

Die laborinterne statistische Qualitätskontrolle erfolgt mit einem Kontrollprobensystem, welches zwei verschiedene Anforderungen zu erfüllen hat:
1. Präzisionskontrolle
2. Richtigkeitskontrolle

Für die Kontrolle der Präzision ist es ausreichend, wenn sie an der häufigsten Entscheidungsgrenze erfolgt. Die Kontrolle der Richtigkeit muß über den jeweils klinisch relevanten Bereich der Meßgröße erfolgen. Sie sollte daher auch außerhalb der Norm, im pathologischen Bereich möglich sein.

Unter Richtigkeit versteht man die Übereinstimmung des Analysenergebnisses mit dem "wahren Wert". Es gibt demzufolge für eine Meßgröße in einer Probe nur einen einzigen Referenzmethoden-Wert, aber viele methodische Sollwerte. Eine unabdingbare Voraussetzung zur Ermittlung eines wahren Wertes des Analyten in einem Kontrollserum ist daher der Umstand, daß der Analyt im Kontrollmaterial möglichst genau die Form, Struktur und Art des Stoffes widerspiegelt, in der er auch nativ in der Patientenprobe vorliegt.

Bei der Präzisionskontrolle sollte dasselbe Kontrollmaterial über einen möglichst langen Zeitraum eingesetzt werden. Diese Bedingung setzt eine hohe Stabilität der Meßgröße im verwendeten Kontrollmaterial voraus. Für jede Analytenserie muß mindestens eine Präzisionskontrollprobe analysiert werden. Liegt das Ergebnis der Kontrollprobe nicht in den erforderlichen Kontrollgrenzen, so muß die Ursache festgestellt und die gesamte Analytenserie wiederholt werden. Die Kontrolle der Richtigkeit hat einen besonders großen Einfluß auf den Prozentsatz der Fehlzuordnungen von Patientenproben und ist somit von größter klinischer Relevanz.

Die Ringversuche stellen die gesetzlich vorgeschriebene externe Richtigkeitskontrolle sicher und unterstützen die interne Richtigkeitskontrolle des medizinischen Laboratoriums. Die Bewertung der Ringversuchsergebnisse erfolgt demzufolge nach denselben Kriterien wie bei der laborinternen Richtigkeitskontrolle.

Prinzipiell gilt, daß sowohl die interne Präzisions- und Richtigkeitskontrolle als auch die externe Richtigkeitskontrolle mit einem Kontrollmaterial durchgeführt werden soll, das weitestgehend allen Verhälnissen und methodisch relevanten Vorgaben des Untersuchungsmaterials der Patienten gleichkommt.

Diese Forderung kann bislang für bestimmte Analyten jedoch nicht oder nur in eingeschränktem Maße, d.h. unbefriedigend erfüllt werden. Dies gilt insbesondere für hochmolekulare Verbindungen oder/und Komplexe, zu deren Synthese vielfältige Zellaktivitäten und Stoffwechselprozesse in bestimmten Organen oder in einem Gesamtorganismus Voraussetzung sind.

Im US-Patent 5,089,602 wird eine Verfahren zur Aufreinigung von Apolipoproteinen aus humanem Plasma oder Serum beschrieben unter Verwendung eines Präzipitationsprozesses. US 5,089,602 beschreibt nicht die Gewinnung eines biologisch nativen und aktiven Produkts, wie ein Lipoprotein, das bei einer Apherese aus Körperflüssigkeiten abgetrennt wird.

WO 98/07751 beschreibt ein Verfahren zur Aufreinigung von Apolipoprotein A oder Apolipoprotein E aus humanem Plasma unter Verwendung eines Anionenaustauscher-Chromatographie-Schritts. Lipoproteine aus einer Körperflüssigkeit durch Apherese zu entfernen und anschließend in eine biologisch native und aktive Form zu überführen, ist in WO 98/07751 nicht beschrieben.

Groß et al. (Protein Expression & Purification 5, (1994), 112-117) beschreibt die Isolierung von Lipoprotein (a) ausgehend von Produkten, welche bei verschiedenen Aphereseverfahren anfallen, wie z.B. Plasma. Groß et al. lehrt, dass nicht alle Aphereseprodukte zur Isolierung von Lipoprotin (a) geeignet sind, das insbesondere bei dem von HELP-System stammenden Material in allen Schritten der Isolierung eine Aggregation auftritt. Zudem ist das in Groß et al. erhaltene Lipoprotein (a) mit LDL verunreinigt.

WO 96/41198 beschreibt ein Verfahren zur Isolierung von großen Mengen an Lipoprotein (a) aus einer biologischen Flüssigkeit. Die Rückgewinnung von Lipoproteinen in biologisch nativer und aktiver Form aus Aphereseprodukten ist nicht beschrieben.

Der Erfindung liegt deshalb die Aufgabe zugrunde, ein Verfahren zur Gewinnung von Lipoproteinen in unveränderter nativer Form bereitzustellen, das für die Herstellung von Proben- bzw. Kontrollmaterialien in flüssiger oder fester, z.B. lyophilisierter Form, bevorzugt auf Plasma- oder Serum-Basis geeignet ist. Dabei sollten die gewonnenen Proben- bzw. Kontrollmaterialien die Kriterien für die klinisch-chemische Qualitätskontrolle (u.a. Richtigkeit, Präzision, Spezifität und Stabilität) erfüllen. Insbesondere sollte die Erfindung die Aufgabe lösen, Proben- bzw. Kontrollmaterialien herzustellen, deren Lipoproteine im oberen Norm- bzw. pathologisch erhöhten Bereich vorliegen. Die Erfindung sollte auch die Aufgabe lösen, Infusions-, Nähr- oder/und Inkubationslösungen bzw. dafür geeignete Fraktionen herzustellen, deren Lipoprotein(e) in nativer bzw. aktiver Form und in hoher Konzentration vorliegen.

Erfindungsgemäß wurde die genannte Aufgabe dadurch gelöst, daß zur Gewinnung der Lipoproteine vorzugsweise auf Verfahren zurückgegriffen wird, die als therapeutische Maßnahme bei Patienten eingesetzt werden, um derartige Stoffe aus Körperflüssigkeiten, z.B. dem Blut bzw. Plasma oder Serum der Patienten unter Verwendung eines extrakorporalen Apheresesystems zu eliminieren. Die Abtrennung und erfindungsgemäße Rückgewinnung der Lipoproteine in nativer bzw. aktiver Form kann aber auch aus frisch gewonnenem (gespendeten) oder aus entsprechend konserviertem Blut, Plasma oder Serum erfolgen.

Überraschenderweise wurde festgestellt, daß derartige Stoffe - nachdem diese durch Filtration, Adsorption oder/und Präzipitation aus Körperflüssigkeiten, z.B. Blut, Serum oder Plasma von Menschen oder Tieren, in einem extrakorporalen Trennsystem angefallen sind - in biologisch aktiver Form wieder rückgewonnen und als Konzentrat in nativem Zustand bereitgestellt werden können. Diese Konzentrate können dann wiederum mit Lösungen wie beispielsweise physiologische Kochsalzlösung und/oder Matrices wie beispielsweise Blut, Plasma, Serum, Urin, Liquor und Albuminlösung versetzt werden, um beispielsweise Proben- bzw. Kontrollmaterialien herzustellen, deren Komponenten im oberen Norm- bzw. pathologisch erhöhten Bereich vorliegen.

Die Erfindung betrifft somit ein Verfahren zur Gewinnung von Lipoproteinen, umfassend die Rückgewinnung von Lipoproteinen in biologisch nativer und aktiver Form, die zuvor bei einer Apherese aus einer Körperflüssigkeit, insbesondere Blut oder/und Plasma, abgetrennt worden sind und worin die Rückgewinnung eine Rekonstitution der biologischen Komponenten in einem Puffer umfasst, der ein delipidiertes Albumin enthält. Die Abtrennung erfolgt gemäß Erfindung in einem geeigneten extrakorporalen Trenn- oder Apheresesystem. Die Abtrennung erfolgt vorzugsweise durch Präzipitation und Adsorption oder Filtration. Ein bevorzugtes Beispiel für ein derartiges extrakorporales Apheresesystem ist das H.E.L.P.-System, welches auf einer weitgehend spezifischen Präzipitation von Low Density-Lipoproteinen (LDL) und Lp(a) aus dem Plasma von behandlungsbedürftigen Patienten in Anwesenheit von Heparin bei einem niedrigen pH-Wert basiert (Klinische Wochenschrift 65, 161-168, 1987). Andere extrakorporale Eliminationssysteme für Lipoproteine beruhen auf einer größenabhängigen Membranfiltration oder einer Adsorption an modifizierte, poröse Trägermaterialien wie beispielsweise Dextransulfat-Cellulose und Sepharose mit immobilisierten Antikörpern gegen Apolipoprotein B-100 (International Journal of Artificial Organs 12, 61-65, 1989) oder ein Copolymer mit Polyacrylsäure-Liganden (EP 0 424 698 B1). Die jeweils anfallenden Filtrate oder Adsorbate können ebenfalls als Ausgangsmaterial verwendet werden.

Beispiele für so zu gewinnende Lipoproteine sind insbesondere Fraktionen, Subfraktionen, Isoformen oder Gemische von Plasmalipoproteinen (siehe Tabelle 1).

Die Rückgewinnung der Lipoproteine umfaßt ihre Rekonstitution in biologisch nativer bzw. aktiver Form. Hierzu wird ein die gewünschten Lipoproteine enthaltendes Präzipitat oder ein mit den gewünschten Lipoproteinen beladener Adsorber oder ein Filtrat mit einem geeigneten Puffer unter Bedingungen in Kontakt gebracht,
die zu einer Rekonstitution der gewünschten Lipoproteine im Puffer in biologisch nativer Form führen. Bei der Gewinnung von Lipoproteinen gemäß der vorliegenden Erfindung wird ein Puffer verwendet, der delipidiertes Albumin, das frei an freien Fettsäuren ist, enthält. Nach der Gewinnung können die Lipoproteine lyophilisiert und bei Bedarf unter Beibehalt ihrer nativen Form bzw. biologischen Akivität in einem geeigneten Medium, z.B. in einer Körperflüssigkeit oder einem Puffer rekonstituiert werden.

Die durch das Verfahren gewonnenen Lipoproteine können beliebigen Verwendungen zugeführt werden. So können beispielsweise bestimmte Lipoproteinfraktionen, bevorzugterweise HDL oder Lp(a) zu therapeutischen Zwecken verwendet werden (vgl. Beispiel 2). Hierzu wird das isolierte Lipoprotein wie beispielsweise Lp(a) einem Patienten infundiert, um protektiv hinsichtlich einer Infektion oder Sepsis zu wirken. Die erfindungsgemäß hochkonzentrierten Lösungen eignen sich ganz besonders für Infusionszwecke, da einerseits die infundierte Menge der Lipoproteine sehr hoch und andererseits das infundierte Volumen der entsprechenden Infusionslösung relativ klein gehalten werden kann. Dadurch kann die Dosierung bzw. therapeutische Effizienz gesteigert werden, ohne daß es zu unerwünschten hämodynamischen Störungen des Blutkreislaufs eines Patienten kommt. Darüberhinaus erlaubt das erfindungsgemäße Verfahren, einem Patienten erstmalig therapeutisch nützliche Lipoproteine vor einer entsprechenden Therapie durch eine oder mehrere extrakorporale Eigenblut-Behandlungen anzureichern und z.B. in Form einer Infusionslösung bereitzustellen. Auch eignen sich derartige hochkonzentrierte Lösungen für die Herstellung von Nähr- und Inkubationsmedien für Zellkulturen (vgl. Beispiel 3). Besonders bevorzugt ist jedoch die Herstellung von Kontrollen oder Standards für diagnostische Tests, z.B. zur Bestimmung von Cholesterin, von jeder Art von Lipoprotein, der (Apo)Lipoproteine A, B, C, E, deren Isoformen, oder zur Bestimmung von Lipiden bestimmter Lipoprotein-Fraktionen. Besonders bevorzugt werden
Kontrollen oder Standards hergestellt, die ein nachzuweisendes Lipoprotein, d.h. den diagnostischen Parameter im oberen Normbereich bzw. im pathologisch erhöhten Bereich enthalten (vgl. Beispiel 4).

Im weiteren soll die vorliegende Erfindung am Beispiel der Gewinnung von Plasmalipoproteinen, insbesondere der LDL-Fraktion mit ihren Untereinheiten, sowie LP(a) aus einem extrakorporalen Blut- oder Plasmaperfusionssystem beschrieben werden. Als besonders bevorzugt hat sich dabei das System der heparininduzierten extrakorporalen Elimination von Plasmaproteinen (H.E.L.P. Therapie) erwiesen.

### Beispiele

### Beispiel 1 Gewinnung von Plasmalipoproteinen

### 1.1 Nomenklatur und Zusammensetzung der Plasmalipoproteine

Die Lipide des Blutplasmas (Cholesterin, Triglyceride und Phospholipide) sind ihrer Natur nach wasserunlöslich und liegen in dem wässrigen Milieu des Blutes durch die Verbindung mit spezifischen Proteinen in einer löslichen Form vor. Diese partikulären Komplexe werden als Plasmalipoproteine bezeichnet.

Das Plasmalipoprotein-Spektrum des Menschen ergibt sich aus den absoluten Konzentrationen und den Konzentrationsverhältnissen verschiedener Plasmalipoprotein-Fraktionen zueinander. Unterschiede zwischen Plasmalipoproteinen können sich z.B. in ihrer Größe ausdrücken. Eng verwandt mit der Größe ist das spezifische Gewicht der Lipoproteine. Unterschiede im spezifischen Gewicht macht man sich für ihre Trennung mit Hilfe der Ultrazentrifuge zunutze. Dieses Trenn- und Analysensystem ist der Ursprung einer noch heute gebräuchlichen Nomenklatur der Lipoproteine
(Chylomikronen; VLDL d < 1.006 g/ml; LDL d = 1.006 - 1.063 g/ml; HDL d = 1.063 - 1.250 g/ml).

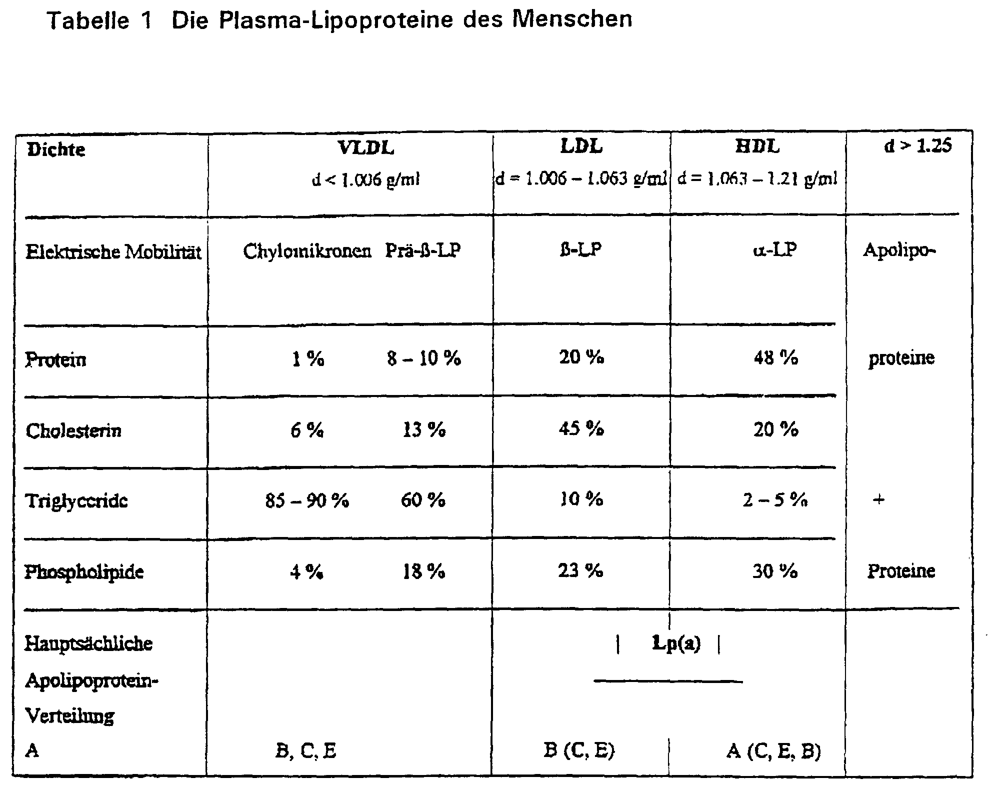

Eine fast gleichberechtigte Nomenklatur beruht auf unterschiedlichen elektrischen Ladungen und entsprechend unterschiedlichen elektrophoretischen Mobilitäten der Plasmalipoproteine. Hier unterscheidet man β-, Prä-β- und α-Lipoproteine. Für Chylomikronen benötigt man keine Ultrazentrifuge, um sie zur Flotation zu bringen, bei der elektrophoretischen Trennung bleiben sie am Start liegen. Im wesentlichen entsprechen sich Dichteklassen und elektrophoretische Banden (siehe Tabelle 1).

### 1.2 Störungen des Lipidstoffwechsels

Von allen Stoffwechselstörungen des Menschen neben dem Diabetes mellitus sind die Hyperlipoproteinämien die häufigsten und von besonderer klinischer Relevanz. Sie gelten als hauptsächlicher Risikofaktor für die Entstehung von kardiovaskulären Erkrankungen und sind damit hauptverantwortlich für die häufigste Todesursache in der Bundesrepublik Deutschland und in anderen Industrieländern. Die Ermittlung der einzelnen Lipoproteinfraktionen in ihrer Konzentration und Qualität ist daher klinisch von überragender Bedeutung. Konzentrationsverschiebungen dieser Fraktionen absolut und zueinander (Hyperlipoproteinämien, Dyslipoproteinämien) lassen sich durch eine alleinige Ermittlung der Blutfette, des Plasmacholesterins, der Triglyceride und der Phospholipide nicht sicher erkennen. Umgekehrt basiert eine fundierte Risikoabschätzung und eine zielgerichtete Therapie auf einer präzisen Analytik der Lipoproteine. So erfordern alle Empfehlungen zur Behandlung von Fettstoffwechselstörungen, insbesondere in bezug auf die Prävention kardiovaskulärer Erkrankungen eine genaue Bestimmung der Konzentration und eine entsprechende therapeutische Zielvorgabe besonders der Low-Density Lipoproteine (LDL) und High Density Lipoproteine (HDL) bzw. deren entsprechenden Cholesterinanteilen. Diesem therapeutischen Interventionskonzept folgend müssen die analytischen Bestimmungsmethoden darauf ausgerichtet sein, die Konzentration der Lipoproteine im Plasma oder Serum präzise und richtig zu erfassen. Dieses gilt insbesondere für den oberen Norm- bzw. pathologischen Bereich. Derzeit stehen verschiedene analytisch-diagnostische Verfahren für die routinemäßige Quantifizierung der Plasmalipoproteine zur Verfügung. Dennoch stehen bislang keine Kontrollmaterialien mit nativen Plasmalipoproteinen im oberen Norm- bzw. pathologisch erhöhten Bereich zur Verfügung. Daher entzieht sich dieser - für die therapeutische Intervention und Verlaufskontrolle entscheidende - Konzentrationsbereich einer adäquaten Richtigkeitskontrolle und damit der Qualitätskontrolle in der Diagnostik und im Therapieverlauf.

Die komplexe Struktur der humanen Plasmalipoproteine setzt eine hohe biologische Syntheseleistung auf zellulärer Ebene und im Gesamtorganismus voraus, die in vitro oder mit Hilfe rekombinanter Techniken bisher nicht nachvollzogen werden kann. Auch lassen sich hohe Konzentrationen von Lipoproteinen nicht durch die Aufstockung einzelner Lipide, z.B. Cholesterin, Triglyceride oder Phospholipide erreichen, da die Lipide als wasserunlösliche Stoffe zur Trübung solcher Kontrollmaterialien führen, was die Bestimmung einer großen Anzahl anderer Analyten beeinflußt oder unmöglich macht. Umgekehrt steht die Verwendung von Plasma oder Serum von Patienten, die an einer Hyper- oder Dyslipoproteinämie leiden, quantitativ nicht in ausreichender Menge zur Verfügung, als daß sie zur Herstellung von Qualitätskontrollmaterialien verwendet werden könnten.

### 1.3 Die H.E.L.P. Behandlung

Das Prinzip der H.E.L.P.-Behandlung basiert auf einer spezifischen Präzipitation der Low-Density-Lipoproteine und des Lp(a) aus dem Plasma von behandlungsbedürftigen Patienten in Anwesenheit von Heparin bei niedrigem pH-Wert.

In einem ersten Schritt werden die Blutzellen von dem Plasma mit Hilfe eines Plasmaseparators getrennt. Die Blutzellen werden dem Patienten sogleich zurückgeführt, das Plasma wird kontinuierlich mit einem 0,3 mol/l Acetatpuffer, der Heparin enthält, bei pH 4,85 vermischt. Bei dem sich einstellenden pH von 5,12 kommt es zu einer sofortigen Präzipitation der LDL, der Subklassen von LDL, von Lp(a) sowie von Fibrinogen. Die Suspension wird über einen 0.4 *µ*m Polycarbonatfilter geleitet, um das Präzipitat zurückzuhalten. Das verbleibende Plasma bzw. Serum wird dann mit Hilfe einer Ionenaustauschersäule von überschüssigem Heparin befreit, sowie durch eine Ultrafiltration mit Dialyse auf physiologische Verhältnisse zurückgeführt, bevor es dem Patienten vermischt mit den Blutzellen zurückgegeben wird. Während einer Behandlung werden in der Regel drei Liter Plasma prozessiert. Dieses führt zu einer Elimination von ca. 60% der entsprechenden im Blut zirkulierenden Plasmalipoproteine. Diese Menge reicht aus, um z.B. 0.5 bis 1.5 Liter Humanplasma auf die Konzentrationen im oberen Norm- bzw. pathologischen Bereich von Lipoproteinen aufzustocken.

### 1.4 Gewinnung von LDL und Lp(a) Fraktionen in nativer Form

Die nach der H.E.L.P.-Behandlung eines Patienten anfallende Kartusche, die das Präzipitat der aus dem Plasma eliminierten Lipoproteine enthält, wird mit einem geeigneten Puffer eluiert. Vorzugsweise beträgt der pH-Wert des Puffers zwischen 5 und 9, wobei ein pH-Wert von 7,5-8 besonders bevorzugt ist. Die Molarität des Puffers liegt vorzugsweise zwischen 0.02 und 0.50 mol/l, besonders bevorzugt 0.1 mol/l. Als Puffersalz ist beispielsweise Tris-HCl geeignet. Weiterhin enthält der Puffer delipidiertes fettsäurefreies Albumin. Der Gewichtsanteil des Proteins liegt vorzugsweise im Bereich von 0.5 bis 5% (w/w) besonders bevorzugt 2.5%. Weiterhin ist bevorzugt, daß der Puffer NaCl in einem Gewichtsanteil von 0.05 und 5.0% (w/w), besonders bevorzugt 1.0% enthält. Für die Elution wird günstigerweise ein möglichst geringes Puffervolumen von z.B. ≤ 300 ml pro Kartusche verwendet. Die Elution erfolgt vorzugsweise unter rezirkulierenden Bedingungen.

Das Eluat wird zur Stabilitätssteigerung mit EDTA und Natriumazid versetzt und kann anschließend in einer dem Fachmann bekannten Weise weiter sequenziell ultrazentrifugiert oder readsorbiert, repräzipitiert oder refiltriert werden, um die gewünschten Plasmafraktionen zu erhalten. Diese optionalen Reinigungsschritte können auch zur weiteren Abtrennung von copräzipitierten bzw. coeluierten Plasmaproteinen sowie der zusätzlichen Aufkonzentrierung der Fraktionen dienen.

Den auf diese Weise gewonnenen Lipoproteinen bzw. Lipoproteinfraktionen wird anschließend zur Stabilisierung erneut ein lipidfreies Protein, z.B. delipidiertes Albumin zugesetzt, wobei dessen Gewichtsanteil bevorzugterweise zwischen 0.5 und 5% (w/w), besonders bevorzugt bei etwa 2.5% liegt. Anschließend können die Lipoproteine einer Dialyse, z.B. gegen einen 0.9% NaCl/Trispuffer pH 7.4 in Gegenwart von EDTA und Natriumazid unterzogen werden und/oder lyophilisiert werden.

### Beispiel 2 Herstellung von Infusionslösungen für therapeutische Zwecke

Die erfindungsgemäß gewonnenen Lipoproteine wie beispielsweise Lp(a) oder HDL sind geeignet, um Infusionslösungen für therapeutische Zwecke herzustellen, wobei sich derartige Lösungen durch einen sehr hohen Gehalt an Lipoprotein(en) wie z.B. HDL oder Lp(a) auszeichnen. Zu diesem Zweck wird einer physiologischen Kochsalzlösung oder einer anderen geeigneten und dem Fachmann für Infusionszwecke bekannten Lösung das entsprechende Lipoprotein wie z. B. HDL oder Lp(a) in gelöster oder lyophilisierter Form zugesetzt. Die eingesetzten bzw. hergestellten Lösungen werden in der dem Fachmann bekannten Weise, beispielsweise durch Filtration über geeignete Membranen sterilisiert. Als therapeutischer Ansatz kommt ein HDL-Mangel oder auch septische Zustände in Frage.

### Beispiel 3 Herstellung von Lösungen für die Präparation von Nährund/oder Inkubationsmedien

Die erfindungsgemäß gewonnenen Lipoproteine sind geeignet, um Nährund/oder Inkubationslösungen für biologische Zwecke wie beispielsweise der Kultivierung von Zellen herzustellen, wobei sich derartige Lösungen durch einen sehr hohen Gehalt an Lipoproteinen bzw. deren Bestandteile (Cholesterin, Triglyceride, Phospholipide) auszeichnen.

Zu diesem Zweck wird einer physiologischen Kochsalzlösung oder einer anderen geeigneten und dem Fachmann bekannten Nähr- und/oder Inkubationslösung das/die entsprechende(n) Lipoprotein(e) in gelöster oder lyophilisierter Form zugesetzt.

### Beispiel 4 Herstellung von Standard- und Kontrollproben

Die erfindungsgemäß gewonnenen Lipoproteine, z.B. die Lipoproteinfraktionen LDL oder Lp(a) (vgl. Beispiel 1) sind geeignet, um Kontroll- oder Standardmaterialien für diagnostische Tests herzustellen. Hierzu kann eine künstlich hergestellte Probenmatrix, z.B. eine physiologische Kochsalzlösung, eine Albuminlösung,eine Pufferlösung, eine depletierte biologische Flüssigkeit oder eine natürliche Probenmatrix wie z.B. Blut, Plasma, Serum, Urin oder Liquor mit den isolierten Lipoproteinen aufgestockt werden.

So konnte beispielsweise ein Serumpool mit LDL-Cholesterin bis zu einer Konzentration über 1000 mg/l oder mit LP(a) bis zu einer Konzentration mit bis zu über 300 mg/l aufgestockt werden. Zur Aufstockung konnten auch lyophilisierte Produkte eingesetzt werden. Die auf diese Weise hergestellten Standards und Kontrollen mit einem hohen Gehalt an LDL-Cholesterin, bzw. LP(a) weisen keinerlei Trübung auf und sind daher hervorragend zur Durchführung diagnostischer Tests geeignet. Gelelektrophoretische Untersuchungen zeigten weiterhin, daß die zugesetzten Lipoproteine in nativer Form vorliegen. Es konnten keine Abbauprodukte gefunden werden.

## Patentansprüche

1. Verfahren zur Gewinnung von Lipoproteinen, umfassend die Rückgewinnung von Lipoproteinen in biologisch nativer und aktiver Form, die zuvor bei einer Apherese aus einer Körperflüssigkeit abgetrennt worden sind, worin die Rückgewinnung eine Rekonstitution der Lipoproteine in einem Puffer umfasst, der ein delipidiertes Albumin enthält.

2. Verfahren nach Anspruch 1,
**dadurch gekennzeichnet,**
**dass** die Abtrennung eine Präzipitation, eine Adsorption oder/und eine Filtration umfasst.

3. Verfahren nach Anspruch 1 oder 2,
**dadurch gekennzeichnet,**
**dass** die Körperflüssigkeit aus Blut, Plasma oder Serum ausgewählt wird, die aus einem Menschen oder einem Tier stammen können.

4. Verfahren nach einem der vorhergehenden Ansprüche 1 bis 3,
**dadurch gekennzeichnet,**
**dass** man eine frisch gewonnene oder konservierte Körperflüssigkeit verwendet.

5. Verfahren nach einem der Ansprüche 1 bis 4,
**dadurch gekennzeichnet,**
**dass** die Abtrennung in einem extrakorporalen System erfolgt.

6. Verfahren nach einem der Ansprüche 1 bis 5,
**dadurch gekennzeichnet,**
**dass** die Lipoproteine Plasmalipoproteine - VLDL, LDL, HDL, LP(a) - oder Gemische aus diesen in biologisch nativer Form umfassen.

7. Verfahren nach einem der Ansprüche 1 bis 5,
**dadurch gekennzeichnet,**
**dass** Isoformen der Lipoproteine isoliert werden.

8. Verfahren nach einem der vorhergehenden Ansprüche, weiterhin umfassend das Lyophilisieren der Lipoproteine.

9. Verfahren nach Anspruch 8, weiterhin umfassend das Rekonstituieren der lyophilisierten Lipoproteine in einem geeigneten Medium.

10. Verwendung der durch das Verfahren nach einem der Ansprüche 1 bis 9 gewonnenen Lipoproteine zur Herstellung von Kontrollen oder Standards für diagnostische Tests.

11. Verwendung nach Anspruch 10 zur Herstellung von Kontrollen oder Standards zur Bestimmung von Lipoproteinen, Cholesterin, Triglyceriden und Phospholipiden sowie deren Fettsäuremuster.

12. Verwendung nach Anspruch 10 zur Herstellung von Kontrollen oder Standards zur Bestimmung von (Apo) Lipoproteinen, bevorzugt der (Apo)Lipoproteine A, B, C, E, und D und Isoformen davon.

13. Verwendung der durch das Verfahren nach einem der Ansprüche 1 bis 9 gewonnenen Lipoproteine zur Herstellung von Nähr- oder/und Inkubationsmedien für Zellkulturen.

14. Verwendung der durch das Verfahren nach einem der Ansprüche 1 bis 9 gewonnenen Lipoproteine zur Herstellung eines Mittels für therapeutische Zwecke.

15. Verwendung nach Anspruch 14,
**dadurch gekennzeichnet,**
**dass** man eine Infusionslösung herstellt.

16. Verwendung nach Anspruch 15,
**dadurch gekennzeichnet,**
**dass** die Infusionslösung Lipoproteine, insbesondere HDL oder/und Lp(a) zur Prävention oder/und Behandlung einer Infektion oder Septicämie oder eines HDL-Mangels, enthält.

## Claims

1. Process for obtaining lipoproteins comprising the recovery of lipoproteins in a biologically native and active form which have previously been separated from a body fluid in an apheresis, wherein the recovery comprises reconstituting the lipoproteins in a buffer which contains a delipidated albumin.

2. Process as claimed in claim 1,
**characterized in that**,
the separation comprises a precipitation, an adsorption or/and a filtration.

3. Process as claimed in claim 1 or 2,
**characterized in that**
the body fluid is selected from blood, plasma or serum which can be derived from a human or an animal.

4. Process as claimed in one of the previous claims 1 to 3
**characterized in that**
a freshly collected or preserved body fluid is used.

5. Process as claimed in one of the claims 1 to 4,
**characterized in that**
the separation occurs in an extracorporeal system.

6. Process as claimed in one of the claims 1 to 5,
**characterized in that**
the lipoproteins comprise the plasma lipoproteins - VLDL, LDL, HDL, Lp(a) - or mixtures thereof in a biologically native form.

7. Process as claimed in one of the claims 1 to 5,
**characterized in that**
isoforms of the lipoproteins are isolated.

8. Process as claimed in one of the previous claims, additionally comprising lyophilization of the lipoproteins.

9. Process as claimed in claim 8, additionally comprising reconstitution of the lyophilized lipoproteins in a suitable medium.

10. Use of the lipoproteins obtained by the process as claimed in one of the claims 1 to 9 to produce controls or standards for diagnostic tests.

11. Use as claimed in claim 10 for producing controls or standards for determining of lipoproteins, cholesterol, triglycerides and phospholipids and their fatty acid patterns.

12. Use as claimed in claim 10 to produce controls or standards for determining (apo) lipoproteins, preferably the (apo) lipoproteins A, B, C, E and D and isoforms thereof.

13. Use of the lipoproteins obtained by the process as claimed in one of the claims 1 to 9 to produce nutrient and/or incubation media for cell cultures.

14. Use of the lipoproteins obtained by the process as claimed in one of the claims 1 to 9 to produce an agent for therapeutic purposes.

15. Use as claimed in claim 14,
**characterized in that**
an infusion solution is produced.

16. Use as claimed in claim 15,
**characterized in that**
the infusion solution contains lipoproteins, in particular HDL or/and Lp(a) to prevent or/and treat an infection or septicaemia or a HDL deficiency.

## Revendications

1. Procédé d'obtention de lipoprotéines, comprenant la récupération de lipoprotéines sous forme biologiquement native et active qui ont été séparées au préalable d'un liquide corporel lors d'une aphérèse, la récupération comprenant une reconstitution des lipoprotéines dans un tampon qui contient une albumine délipidée.

2. Procédé selon la revendication 1,
**caractérisé en ce que**
la séparation comporte une précipitation, une adsorption et/ou une filtration.

3. Procédé selon la revendication 1 ou 2,
**caractérisé en ce qu'**
on choisit le liquide corporel parmi le sang, le plasma ou le sérum qui peuvent provenir d'un être humain ou d'un animal.

4. Procédé selon l'une quelconque des revendications précédentes 1 à 3,
**caractérisé en ce qu'**
on utilise un liquide corporel fraîchement obtenu ou conservé.

5. Procédé selon l'une quelconque des revendications 1 à 4
**caractérisé en ce que**
la séparation se déroule dans un système extracorporel.

6. Procédé selon l'une quelconque des revendications 1 à 5,
**caractérisé en ce que**
les lipoprotéines englobent des lipoprotéines plasmatiques - VLDL, LDL, HDL, LP(a) - ou des mélanges de celles-ci sous forme biologiquement native.

7. Procédé selon l'une quelconque des revendications 1 à 5,
**caractérisé en ce qu'**
on isole des formes iso des lipoprotéines.

8. Procédé selon l'une quelconque des revendications précédentes, comportant en outre la lyophilisation des lipoprotéines.

9. Procédé selon la revendication 8, comportant en outre la reconstitution des lipoprotéines lyophilisées dans un milieu approprié.

10. Utilisation des lipoprotéines obtenues par le procédé selon l'une quelconque des revendications 1 à 9 pour fabriquer des témoins ou des standards destinés à des tests diagnostiques.

11. Utilisation selon la revendication 10 pour fabriquer des témoins ou des standards destinés à la détermination de lipoprotéines, de cholestérol, de triglycérides et de phospholipides ainsi que leurs modèles d'acide gras.

12. Utilisation selon la revendication 10 pour fabriquer des témoins ou des standards destinés à la détermination de lipoprotéines (Apo), de préférence des lipoprotéines (Apo) A, B, C, E et D et les formes iso de celles-ci.

13. Utilisation des lipoprotéines obtenues par le procédé selon l'une quelconque des revendications 1 à 9 pour fabriquer des milieux de culture et/ou des milieux d'incubation pour des cultures de cellules.

14. Utilisation des lipoprotéines obtenues par le procédé selon l'une quelconque des revendications 1 à 9 pour fabriquer un agent à des fins thérapeutiques.

15. Utilisation selon la revendication 14,
**caractérisée en ce qu'**
on fabrique une solution pour perfusion.

16. Utilisation selon la revendication 15,
**caractérisée en ce que**
la solution pour perfusion contient des lipoprotéines, en particulier des HDL et/ou des Lp(a) afin de prévenir et/ou de traiter une infection ou une septicémie ou bien une carence en HDL.
